# EUROPEAN PATENT APPLICATION

(11) **EP 4 844 331 A1**
(43) Date of publication of application: **25.11.2026**
(21) Application number: 25178150.6
(22) Date of filing: 22.05.2025
(51) Int. Cl.: G01B 9/02055, G01B 9/02091, A61B 3/10

(54) **DELAY LINE ADJUSTMENT IN AN OCT IMAGING INSTRUMENT**

(71) Applicant: Optos plc, Dunfermline, Scotland KY11 8GR (GB)
(72) Inventor: EVANS, David, Dunfermline, Scotland, KY11 8GR (GB); BUSTIN, Nicholas, Dunfermline, Scotland, KY11 8GR (GB); MUYO, Gonzalo, Dunfermline, Scotland, KY11 8GR (GB); KASAI, Hiroshi, Dunfermline, Scotland, KY11 8GR (GB); MIZUTA, Masahiro, Tokyo, 108-6290 (JP)
(74) Representative: Hoffmann Eitle

(57) **Abstract**

An OCT instrument for imaging a portion of an imaging target, comprising: an interferometer comprising a reference arm and a sample arm having an optical element; an optical delay line for adjusting an optical delay between the interferometer arms; a detector which generates a signal indicative of an interference light from the interferometer; and a controller which changes the optical delay by: controlling the optical delay line to start varying the optical delay; generating A-scans by processing the signal such that each A-scan corresponds to a different optical delay; processing each A-scan to determine whether it images a portion of the optical element, until a first A-scan which images the portion is processed; and in response to processing the first A-scan, controlling the optical delay line to change the optical delay by a predetermined amount to a target value for imaging the imaging target.

## Description

### [Field]

Example aspects herein generally relate to the field of ophthalmic imaging instruments and, more particularly, to arrangements and methods of adjusting a delay line provided in an interferometer of an optical coherence tomography (OCT) imaging instrument.

### [Background]

Optical coherence tomography (OCT) is an imaging technique based on low-coherence interferometry, which is widely used to acquire high-resolution two- and three-dimensional images of optical scattering media, such as biological tissue.

OCT imaging instruments can be classified as being time-domain OCT (TD-OCT) or Fourier-domain OCT (FD-OCT) (also referred to as frequency-domain OCT). In TD-OCT, an optical path length of a reference arm of the imaging instrument's interferometer is varied in time during the acquisition of a reflectivity profile of the scattering medium being imaged by the OCT imaging system (referred to herein as the "imaging target"), the reflectivity profile being commonly referred to as a "depth scan" or "axial scan" ("A-scan"). In FD-OCT, a spectral interferogram resulting from an interference between light in the reference arm and light in the sample arm of the interferometer at each A-scan location is Fourier transformed to simultaneously acquire all points along the depth of the A-scan, without requiring any variation in the optical path length of the reference arm. FD-OCT can allow much faster imaging than scanning of the sample arm mirror in the interferometer, as all the back-reflections from the sample are measured simultaneously. Two common types of FD-OCT are spectral-domain OCT (SD-OCT) and swept-source OCT (SS-OCT). In SD-OCT, a broadband light source delivers many wavelengths to the imaging target, and all wavelengths are measured simultaneously using a spectrometer as the detector. In SS-OCT (also referred to as time-encoded frequency-domain OCT), the light source is swept through a range of wavelengths, and the temporal output of the detector is converted to spectral interference.

An adjustable optical delay line (also referred to as a "variable" or "tuneable" optical delay line) is normally provided in the sample arm or the reference arm of the interferometer forming part of the OCT imaging instrument, which may be of any of the kinds set our above. The adjustable optical delay line is used to set the location of the range of distances from the OCT imaging instrument over which the OCT image is acquired by the instrument, in other words the location of the so-called "OCT imaging window" in front of the instrument that needs to overlap the imaging target. The adjustable optical delay line is typically of a free-space type, and comprises a retroreflector mounted on a motorised linear translation stage, which is moveable to vary an optical path length of light propagating through the adjustable optical delay line. The motorised translation stage may be driven by any appropriate means, for example a piezo transducer in cases where a relatively small travel range is required, or a motor such as a slip-stick motor, an inchworm motor or a DC motor with an encoder in cases where a larger travel range is required.

Motorised translation stages and other mechanisms that are often employed in adjustable optical delay lines tend to introduce some variability in the optical delay which is achieved when the adjustable optical delay line is repeatedly adjusted back to the same target setting. This variability depends on the type of optical delay line used, and may be reduced by taking measures such as equipping the optical delay line with an optical interferometer and a control mechanism which uses the interferometer to accurately monitor movements within the delay line and correct for deviations from target values using a feedback loop. However, such measures add complexity and additional maintenance requirements, as well as cost, and yet still fail to account for unwanted changes to the delay between the arms of the OCT imaging instrument's interferometer that may originate outside the optical delay line.

### [Summary]

There is provided, in accordance with a first example aspect herein, an optical coherence tomography (OCT) imaging instrument arranged to acquire an OCT image of an imaging target, such as a portion of an eye. The OCT imaging instrument may, for example, be an ophthalmic OCT imaging instrument arranged to acquire an OCT image of a portion of an eye The OCT imaging instrument comprises an interferometer having a reference arm and a sample arm comprising an optical element, the interferometer being arranged to generate an interference light during use of the OCT imaging instrument by combining a sample light that has been scattered by the imaging target and propagates via the optical element and along the sample arm during acquisition of the OCT image, with a reference light propagating along the reference arm during acquisition of the OCT image. The OCT imaging instrument further comprises an adjustable optical delay line, which is controllable to adjust an optical delay between the sample light and the reference light, and a detector arranged to detect the interference light and generate an electrical signal indicative of the detected interference light. The OCT imaging instrument further comprises a controller arranged to control the adjustable optical delay line, using the electrical signal, to change the optical delay to a target value for imaging the imaging target, by: controlling the adjustable optical delay line to start varying the optical delay; after the adjustable optical delay line has started to vary the optical delay, generating A-scans by processing the electrical signal being generated by the detector, such that each of the A-scans corresponds to a different value of the optical delay; processing each A-scan to determine whether A-scan data in a portion of the A-scan is indicative of a portion (e.g. surface) of the optical element having been imaged in the portion of the A-scan, until a first A-scan comprising A-scan data that is indicative of the portion of the optical element having been imaged in the portion of the A-scan has been processed; and in response to the processing of the first A-scan, controlling the adjustable optical delay line to change the optical delay by a predetermined amount to the target value for imaging the imaging target, the predetermined amount being dependent on an optical path length of the sample light between the imaging target and the optical element when the imaging target is disposed at a predetermined position relative to the OCT imaging instrument for the OCT image to be acquired.

In some example embodiments, the OCT imaging instrument may further comprise an objective lens system provided in the sample arm of the interferometer, and at least one scanning element arranged to perform a scan of a beam of light across the imaging target via the objective lens system during acquisition of the OCT image, the objective lens system being arranged such that the scan has a non-rectangular scan geometry. The objective lens system may, for example, be arranged such that the scan has a converging (e.g. concentric) scan geometry.

Furthermore, in the OCT imaging instrument of the above example embodiments, the optical element may comprise a lens, and the objective lens system may comprise a plurality of lenses that includes the lens, the lens being an outermost lens of the plurality of lenses which is closest to the imaging target during acquisition of the OCT image during use of the OCT imaging instrument to image the imaging target. The portion of the optical element may, for example, be a surface of the outermost lens, which surface faces the imaging target during acquisition of the OCT image.

In the OCT imaging instrument of the first example aspect or any of its example embodiments set out above, the imaging target may comprise an anterior segment of an eye, and the OCT imaging instrument may be further arranged to acquire an OCT image of a retina of the eye, the controller being arranged to control the adjustable optical delay line, using the electrical signal, to change the optical delay from a predetermined value, which is for imaging the retina of the eye, to the target value for imaging the anterior segment of the eye.

In example embodiments where the OCT imaging instrument further comprises an objective lens system provided in the sample arm of the interferometer, and at least one scanning element arranged to perform a scan of a beam of light across a portion of the eye via the objective lens system during acquisition of the OCT image, and where the objective lens system is arranged such that the scan has a non-rectangular scan geometry (e.g. a converging (for example, concentric or arc) scan geometry, or a divergent (e.g. sector) scan geometry), the portion of the eye may comprises an anterior segment of the eye, and the OCT imaging instrument may be further arranged to acquire an OCT image of a retina of the eye, and the controller may be arranged to control the adjustable optical delay line, using the electrical signal, to change the optical delay from a predetermined value, which is for imaging the retina of the eye, to the target value for imaging the anterior segment of the eye. Furthermore, the ophthalmic OCT imaging instrument may further comprise a drive mechanism operable to move the objective lens system along an axis e.g. an optical axis of the objective lens system), towards and away from the eye, and the controller may be further arranged to control the drive mechanism, after controlling the adjustable optical delay line to change the optical delay to the target value for imaging the anterior segment of the eye, to adjust a position of the objective lens system relative to the eye, by: controlling the drive mechanism to start moving the objective lens system along the axis; after the drive mechanism has started to move the objective lens system, generating A-scans by processing the electrical signal being generated by the detector, such that each of the A-scans corresponds a different distance of the objective lens system from the eye; processing each A-scan to determine whether A-scan data in a portion of the A-scan is indicative of a predetermined anatomical feature in the anterior segment having been imaged in the portion of the A-scan, until a first A-scan comprising A-scan data that is indicative of the predetermined anatomical feature having been imaged in the portion of the A-scan has been processed; and in response to the processing of the first A-scan, controlling the drive mechanism to stop moving the objective lens system.

In this case, after the drive mechanism has started to move the objective lens system, the controller may be arranged to generate B-scans of the anterior segment of the eye by processing the electrical signal being generated by the detector, such that each of the B-scans corresponds a different distance of the objective lens system from the eye, and the predetermined anatomical feature may comprise one of a scleral spur of the eye, an iris of the eye, and a predetermined portion (e.g. apex) of a cornea of the eye. Furthermore, the controller may be arranged to process each B-scan to determine whether B-scan data in the B-scan is indicative of the predetermined anatomical feature having been imaged within a predetermined range of positions along an axial direction in the B-scan, until a first B-scan comprising B-scan data that is indicative of the predetermined anatomical feature having been imaged within the predetermined range of positions has been processed, and, in response to the processing of the first B-scan, the controller may be arranged to control the drive mechanism to stop moving the objective lens system.

There is provided, in accordance with a second example aspect herein, a computer-implemented method of generating control signals for controlling an optical coherence tomography (OCT) imaging instrument which is arranged to acquire an OCT image of a portion of an eye. The OCT imaging instrument may be an ophthalmic OCT imaging instrument arranged to image a portion of an eye, and comprises: an interferometer having a reference arm and a sample arm comprising an optical element, the interferometer being arranged to generate an interference light during use of the ophthalmic OCT imaging instrument by combining a sample light that has been scattered by the imaging target and propagates via the optical element, along the sample arm, during acquisition of the OCT image, with a reference light propagating along the reference arm during acquisition of the OCT image; an adjustable optical delay line controllable to adjust an optical delay between the sample light and the reference light; and a detector arranged to detect the interference light and generate an electrical signal indicative of the detected interference light. The method comprise generating control signals for controlling the adjustable optical delay line to change the optical delay to a target value for imaging the imaging target, by: generating a first control signal for controlling the adjustable optical delay line to start varying the optical delay; after the adjustable optical delay line has started to vary the optical delay, generating A-scans by processing the electrical signal generated by the detector, such that each of the A-scans corresponds to a different value of the optical delay; processing each A-scan to determine whether A-scan data in a portion of the A-scan is indicative of a portion (e.g. a surface) of the optical element having been imaged in the portion of the A-scan, until a first A-scan comprising A-scan data that is indicative of the portion of the optical element having been imaged in the portion of the A-scan has been processed; and in response to the processing of the first A-scan, generating a second control signal for controlling the adjustable optical delay line to change the optical delay by a predetermined amount to the target value for imaging the imaging target, the predetermined amount being dependent on an optical path length of the sample light between the imaging target and the optical element when the imaging target is disposed at a predetermined position relative to the OCT imaging instrument for acquiring the OCT image.

In some example embodiments, the OCT imaging instrument may further comprise: an objective lens system provided in the sample arm of the interferometer; and at least one scanning element arranged to perform a scan of a beam of light across the imaging target via the objective lens system during acquisition of the OCT image, the objective lens system being arranged such that the scan has a non-rectangular scan geometry, e.g. a converging (for example, concentric or arc) scan geometry, or a divergent (e.g. sector) scan geometry. Additionally or alternatively, the imaging target may comprise an anterior segment of an eye, the OCT imaging instrument may be further arranged to acquire an OCT image of a retina of the eye, and the generated control signals may be suitable for controlling the adjustable optical delay line to change the optical delay from a predetermined value, which is for imaging the retina of the eye, to the target value for imaging the anterior segment of the eye.

In the latter case, and where the OCT imaging instrument comprises an objective lens system and at least one scanning element as noted above, the OCT imaging instrument may further comprise a drive mechanism controllable to move the objective lens system along an axis (e.g. an optical axis of the objective lens system), towards and away from the eye, and the computer-implemented method may further comprise, after generating the second control signal, generating at least one drive control signal or controlling the drive mechanism to adjust a position of the objective lens system relative to the eye, by: generating a drive control signal for controlling the drive mechanism to start moving the objective lens system along the axis; after the drive mechanism has started to move the objective lens system, generating A-scans by processing the electrical signal generated by the detector, such that each of the A-scans corresponds a different distance of the objective lens system from the eye; processing each A-scan to determine whether A-scan data in a portion of the A-scan is indicative of a predetermined anatomical feature in the anterior segment having been imaged in the portion of the A-scan, until a first A-scan comprising A-scan data that is indicative of the predetermined anatomical feature having been imaged in the portion of the A-scan has been processed; and, in response to the processing of the first A-scan, causing the drive mechanism to stop moving the objective lens system, for example by modifying the drive control signal or generating a second drive control signal for controlling the drive mechanism to stop moving the objective lens system. In some example embodiments, after the drive mechanism has started to move the objective lens system, B-scans of the anterior segment of the eye are generated by processing the electrical signal generated by the detector, such that each of the B-scans corresponds a different position of the objective lens system relative to the eye, the predetermined anatomical feature comprises one of a scleral spur of the eye, an iris of the eye, and a predetermined portion (e.g. an apex) of a cornea of the eye, each B-scan is processed to determine whether B-scan data in the B-scan is indicative of the predetermined anatomical feature having been imaged within a predetermined range of positions along an axial direction in the B-scan (which may include a position corresponding to a focal plane of the objective lens system set for imaging the anterior segment, until a first B-scan comprising B-scan data that is indicative of the predetermined anatomical feature having been imaged within the predetermined range of positions has been processed, and the drive mechanism is caused to stop moving the objective lens system in response to the processing of the first B-scan.

There is provided, in accordance with a third example aspect herein, a computer program comprising computer-readable instructions that, when executed by a processor, cause the processor to perform a method according to the second example aspect or any of its example embodiments set out above. The computer program may be stored on a non-transitory computer-readable medium or carried by a signal.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an ophthalmic OCT imaging instrument according to an example embodiment herein.
Figure 2 a schematic illustration of an example implementation of the ophthalmic OCT imaging instrument of Figure 1, in which the optical element 40 forms part of an objective lens system 80 for imaging the retina and the anterior segment of an eye.
Figure 3A is a schematic illustration of the objective lens system 80 in Figure 2, in a first state for imaging the posterior segment of an eye.
Figure 3B is a schematic illustration of the objective lens system 80 in Figure 2, in a second state for imaging the anterior segment of an eye.
Figure 4 is a schematic illustrating an example implementation of the controller 70 of Figure 1 in programmable signal processing hardware.
Figure 5 is a flow diagram illustrating a method of generating control signals for controlling the adjustable optical delay line in the example embodiment to change the optical delay to a target value for imaging a portion of the eye.

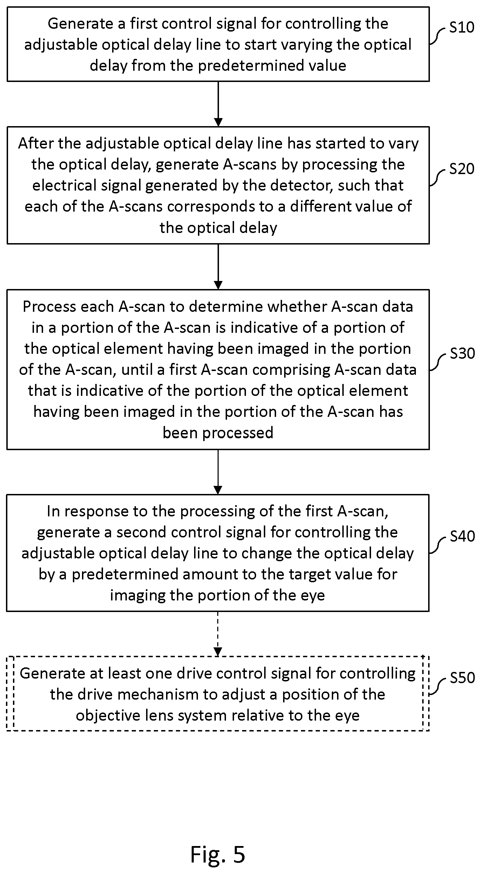

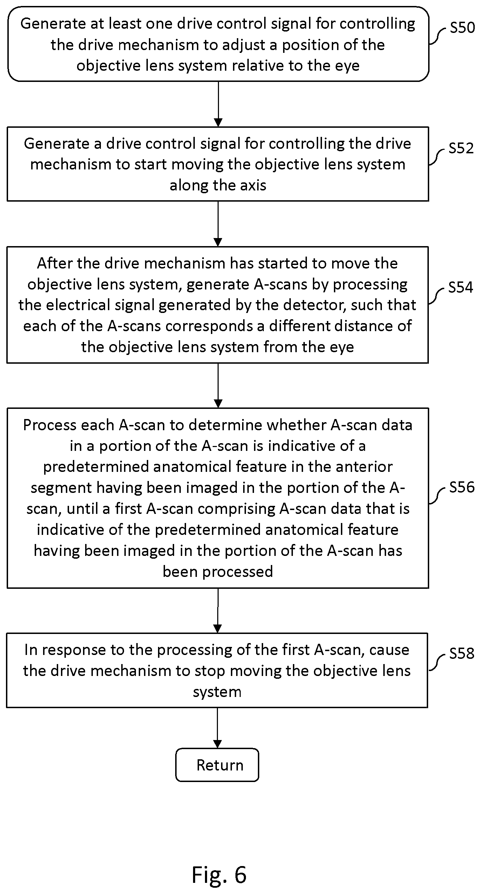

Figure 5 is a flow diagram illustrating an example implementation of optional process S50 in Figure 6.

### [Detailed Description of Example Embodiments]

In view of the shortcomings of conventional OCT imaging instruments discussed above, the inventors have devised methods and hardware for controlling the adjustable optical delay line in the interferometer of an OCT imaging instrument to adjust the position of its OCT imaging window on the basis of OCT data acquired by the instrument as the adjustment is being made, using a surface or another portion of an optical element in the sample arm of the interferometer, which is imaged in the course of the adjustment, as a datum or reference point with respect to which the OCT imaging window is then moved to allow the imaging target to be imaged. These methods and hardware will now be described in more detail with reference to the accompanying figures.

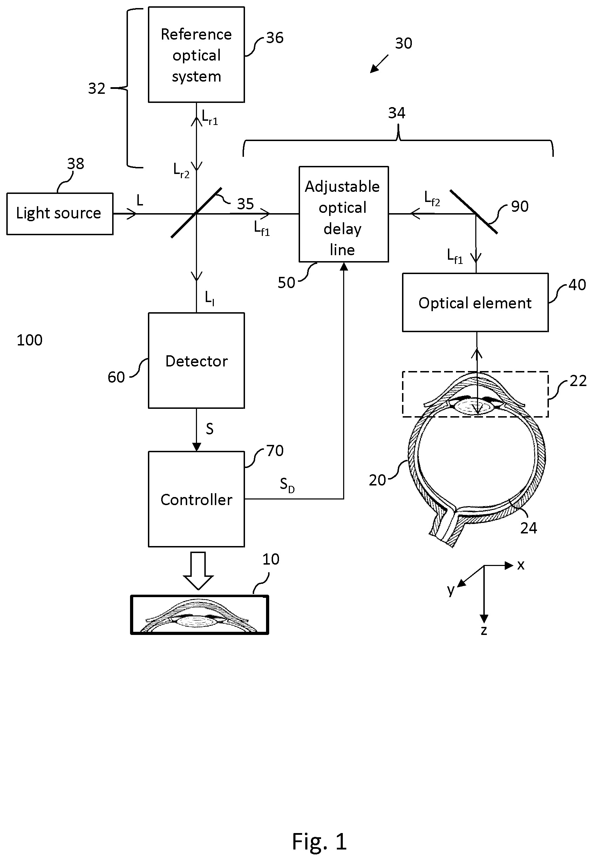

Figure 1 is a schematic illustration of an OCT imaging instrument 100 according to an example embodiment, which is provided in the example form of an ophthalmic OCT imaging instrument arranged to acquire a respective OCT image 10 of each portion of one or more portions of an eye 20. The ophthalmic OCT imaging instrument 100 may, as in the present example embodiment, be operable in an anterior segment (AS) imaging mode to image at least a portion of the anterior segment 22 of the eye 20, the anterior segment 22 comprising the cornea, the angle, the lens, the ciliary body and the iris of the eye 20. The ophthalmic OCT imaging instrument 100 may be further operable in a posterior segment (PS) imaging mode to image a portion of the fundus of the eye 20. The ocular fundus is the inner lining of the eye 20 opposite the lens of the eye 20, and comprises the retina 24, macula, optic disc, fovea, choroid, and blood vessels.

The OCT imaging instrument 100 comprises an interferometer 30 having a reference arm 32 and a sample arm 34. The sample arm 34 comprises an optical element 40, whose role in setting an optical delay between the arms of the interferometer 30 is described below. The interferometer 30 also has optical coupler 35, which may, as in the present example embodiment, be provided in the form of a beam splitter. The beam splitter splits a beam of light L from a light source 38 into two beams. The splitting of the light beam L may be symmetric, such that equal intensity is directed into the resulting two beams, or may be asymmetric, such that unequal intensity is directed into the resulting two beams.

The first beam of light, also referred to herein as a signal light (or sample light, or first light) L_{f1}, propagates along the sample arm 34 of the ophthalmic imaging instrument 100, which has the optical element 40 and may include further optical components, such as scanning element 90, that are arranged to direct the first light L_{f1} to the eye 20 of the subject, and to collect reflected signal light L_{f2} (i.e. a portion of the signal light L_{f1} reflected from the eye 20) and return it to the optical coupler 35. The reflected signal light L_{f2} follows essentially the same path to the optical coupler 35 as the signal light L_{f1} but in a reverse direction.

The second beam, also referred to herein as a reference light Lᵣ₁, is directed by the optical coupler 35 to a reference optical system 36, which is configured to return the reference light, as returning reference light Lᵣ₂, to the optical coupler 35.

The ophthalmic OCT imaging instrument 100 comprises an adjustable optical delay line 50 (also referred to as a variable or tuneable optical delay line), which is controllable by a controller 70 of the ophthalmic OCT imaging instrument 100, as described below, to adjust an optical delay between the reflected signal light L_{f2} and the returning reference light Lᵣ₂. The optical delay line 50 may, as illustrated in Figure 1, be provided in the sample arm 34 of the interferometer 30, although it may be provided in the reference arm 32 in other example embodiments.

At the optical coupler 35, the reflected signal light L_{f2} (i.e. the part of the signal light L_{f1} that has been reflected from the eye 20) and the returning reference light Lᵣ₂ (i.e. the reference light returned from the reference optical system 36) are combined to interfere with one another. The interferometer 30 may thus be arranged to generate the interference light L_{I} during use of the ophthalmic OCT imaging instrument 100 by combining appropriately delayed reflected signal light L_{f2} with the reference light Lᵣ₂ during acquisition of the OCT image 10. The interference light L_{I} is directed to a detector 60, which is arranged to detect the interference light L_{I} and generate an electrical signal S indicative of an intensity of the detected interference light L_{I}.

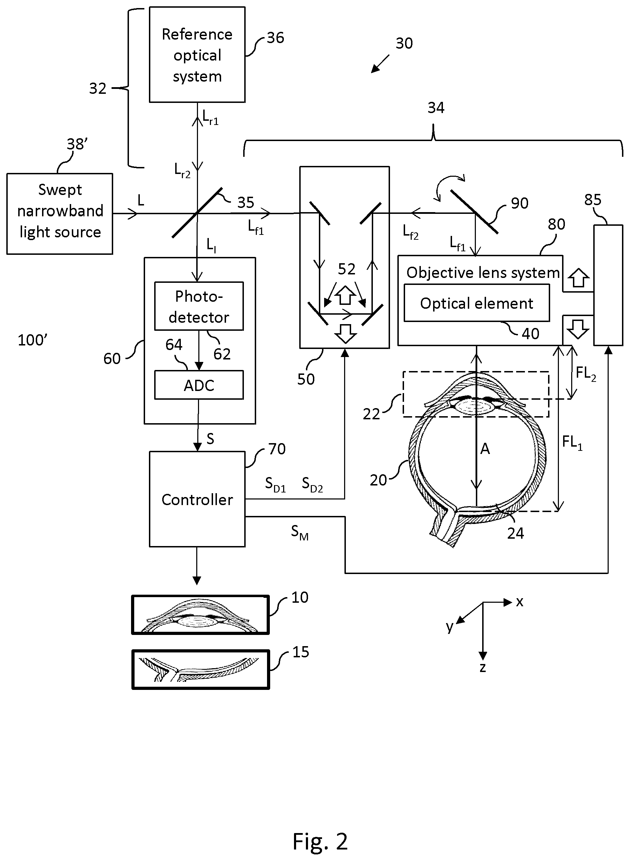

The ophthalmic OCT imaging instrument 100 may be provided in the form of a swept-source OCT (SS-OCT) imaging instrument 100', as illustrated schematically in Figure 2, wherein the light source 38 is provided in the form of a swept narrowband light source 38', and the detector 60 comprises a photodetector 62 and an analog-to-digital converter (ADC) 64.

The swept narrowband light source 38' is arranged to generate a beam of light L, having a varying centre frequency. The swept narrowband light source 38' is configured to vary the centre frequency in a repetitive manner, such as by a repeated periodic frequency sweep over a defined frequency band between a lower frequency and an upper frequency. The sweep may rise in time from low frequency to high frequency, or may fall in time from high frequency to low frequency. The sweep may also successively rise and fall in time. For example, a forward sweep from low to high frequency may be followed by a reverse sweep from high to low frequency.

The swept narrowband light source 38' may be a (wavelength-) swept laser or a swept laser diode, for example an external cavity laser, an optical parametric amplifier, a Fourier-domain mode-locking laser (FDML) or a swept vertical cavity surface-emitting laser (VCSEL), although these examples are not limiting. The bandwidth of the swept narrowband light source 38' may be selected for optimum penetration through, for example, a lens of the eye 20 under examination and tissues of the retina 24 of the eye 20. Also, the bandwidth may, as in the present example embodiment, be in the infra-red region of the optical spectrum, for example, at wavelengths longer than 850 nm.

The photodetector 62 may, for example, comprise a photodiode or an avalanche photodiode, which converts an optical intensity of the interfering beams to provide a resulting converted signal in the form of, e.g. an electrical signal such as a voltage or current. The resulting converted signal may, as in the present example embodiment, be a time-varying analogue signal. After being output by the photodetector 62, the resulting converted signal may be recorded in a time-varying manner, wherein a recorded version of the signal constitutes an interferogram between the reflected signal light L_{f2} and the returning reference light Lᵣ₂ with respect to the optical frequency of the narrowband light from the swept narrowband light source 38'.

The ADC 64 periodically samples and quantises the signal output by the photodetector 62 with a predetermined sample frequency, and then provides digital values of the quantised and sampled signal to the controller (signal processor) 70, wherein the values are recorded. Accordingly, the photodetector 62 and the ADC 64 together constitute the detector 60, which samples the time-varying interference signal. The controller 70 may, as in the present example embodiment, be arranged to perform a Fourier transform operation, such as a fast Fourier transform, on the time-varying quantised and sampled signal values obtained from the ADC 64 to generate an axial depth profile. The axial depth profile may be provided in the form of a one-, two- or three-dimensional data array, depending on whether the measurement beam is scanned across the eye 20 and, if so, the dimensionality of the scan.

As a result of the periodic sampling of the output of photodetector 62 by the ADC 64, the highest-frequency components of the time-varying analogue signal, which lie outside a detection bandwidth of the system, will be aliased, potentially compromising the image. The bandwidth of the system is defined as a combination of electronic bandwidth, which determines the highest frequency that can be detected, and the bandwidth of the ADC 64, which determines the range of frequencies which can be recorded uniquely (i.e. without aliasing). An anti-aliasing filter, which matches the bandwidth of the ADC 64, may be provided between the photodetector 62 and the ADC 64.

The swept narrowband light source 38' may have relatively high coherence length such that the interference between the reflected signal light L_{f2} and the returning reference light Lᵣ₂ contains information about the reflectivity of the retina 24 (or portion of the anterior segment 22 being imaged, as the case may be) over a large axial depth range (i.e. a range of distances along the direction of propagation of the sample light beam L_{f1} in the retina 24) defined by the high coherence length. Nonetheless, only information about a sub-range of this axial depth range, which may correspond to the detection bandwidth of the system or a portion thereof (this subrange being referred to herein as the "OCT imaging window"), which is centred on an axial position defined by the optical path length of the reference arm 32, is available from a Fourier transform of the sampled signal recorded at the controller 70.

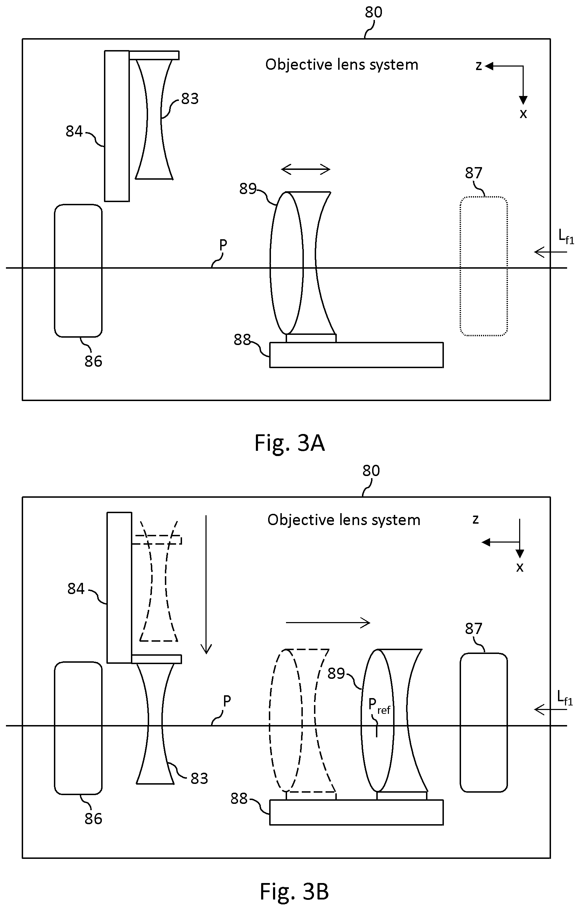

The optical element 40 may, as in the example implementation of Figure 2, form part of an objective lens system (or objective) 80 comprising two or more lenses. In the PS imaging mode, the light source 38' illuminates a portion of the retina 24 with a first light L_{f1} via the objective lens system 80, and the image-forming device formed by the detector 60 and the controller 70 images the illuminated portion of the retina 24 via the objective lens system 80. In the AS imaging mode, the light source 38' illuminates a portion of the anterior segment 22 of the eye 20 with the first light L_{f1} via the objective lens system 80, and the image-forming device images the portion of the anterior segment 22 via the objective lens system 80. To enable the imaging, in turn, of the posterior segment (which includes the retina 24) and the anterior segment 22, the objective lens system 80 has a focal length which is adjustable over at least one range of focal lengths including a first focal length FL₁ for imaging the portion of the retina 24 (but not the anterior segment 22) in the PS imaging mode, and a second focal length FL₂ for imaging the portion of the anterior segment 22 (but not the retina 24) in the AS imaging mode. The focal length may, for example, be adjustable by translation of at least one lens of the objective lens system 80 along an optical axis A of the objective lens system 80, which extends in the z-axis direction shown in Figures 3A and 3B, and optionally also by movement of one of more other lenses into and out of an optical path through the objective lens system 80, as will now be described in more detail with reference to Figures 3A and 3B.

The objective lens system 80 may, as in the present example embodiment, comprise a first focussing optical element 83, and a first drive mechanism 84 arranged to move the first focussing optical element 83 into and out of an optical path P (e.g. of the first light L_{f1}) through the objective lens system 80. Put another way, the first drive mechanism 84 is arranged to move the first focussing optical element 83 (either by translation using rails or the like, as in the present example, or by rotation, for example) between a first position (as shown in Figure 3A) and a second position (as shown in Figure 3B), such that light propagating through the objective lens system 80 does not propagate through the first focussing optical element 83 when it is disposed at the first position but does propagate through the first focussing optical element 83 when it is disposed at the second position. Accordingly, the first focussing optical element 83, when disposed at the second position, contributes to a focussing of light that is performed by the objective lens system 80 and, when disposed at the first position, makes no contribution to the focussing. As will be described in more detail below, for operation in the AS imaging mode, the first drive mechanism 84 is controlled to set the first focussing optical element 83 at the second position shown in Figure 3B for imaging the anterior segment 22 of the eye 20. For operation in the PS imaging mode, the first drive mechanism 84 is controlled to set the first focussing optical element 83 at the first position so that the ophthalmic OCT imaging instrument 100' can image the retina 24 and/or another part of the posterior segment of the eye 20 via the objective lens system 80.

The first focussing optical element 83 may, as in the present example embodiment, comprise a lens of negative refractive power. The first focussing optical element 83 is not so limited, however, and may alternatively comprise a lens of positive refractive power, or another kind of focussing means, such as a Fresnel lens or a Diffractive Optical Element (DOE), for example.

The objective lens system 80 may, as in the present example embodiment, further comprise a first lens group 86 of an overall positive refractive power, and a second lens group 87 of an overall positive refractive power, with the lenses' optical axes coinciding.

The optical element 40 may, as in the present example embodiment, be provided in the form of a lens, which forms part of the first lens group 86 and is preferably the outermost lens of the lenses in the first lens group 86, which lens is closest to the eye 20 and has a surface which faces the eye 20 during acquisition of the OCT image 10. It should be noted, however, that the optical element 40 may be provided in a form other than a lens, for example a non-refracting optical element such as a glass slide or a piece of other substantially transparent material, which may be an outermost optical component of the first lens group 86, which component is closest to the eye 20 and has a surface which faces the eye 20 during acquisition of the OCT image 10. The optical element 40 may in some cases be a transparent dust cover provided between the outermost lens of the first lens group 86 (which lens is closest to the eye 20 during acquisition of the OCT image 10) and the eye 20. However, it should also be noted that, although the optical element 40 is preferably a component of the objective lens system 80 (or another optical component of the ophthalmic OCT imaging instrument 100' that is separate from the objective lens system 80) which is closest to the eye 20 during imaging, in order to reduce the necessary adjustment range of the adjustable optical delay line 50 and the potential for related positioning errors, the optical element 40 may more generally be any optical component of the objective lens system 80, for example the first focussing optical element 83, the second focussing optical element 89 or a lens of the second lens group 87 shown in Figure 3A and 3B and discussed in more detail below, or another optical component of the ophthalmic OCT imaging instrument 100' which is separate from the objective lens system 80 and not the closest of the optical components to the eye 20 during acquisition of the OCT image 10.

The first focussing optical element 83 may be provided in the optical path P between the first lens group 86 and the second lens group 87 when the first focussing optical element 83 is disposed in the second position, as illustrated in Figure 3B. The first focussing optical element 83 may alternatively be disposed on the other side of the second lens group 87 in the optical path P, specifically between the second lens group 87 and the scanning element 90, when the first focussing element 83 is in the second position.

The objective lens system 80 may, as in the present example embodiment, further comprise a second drive mechanism 88 and a second focussing optical element 89, whose position along the optical path P through the objective lens system 80 is adjustable by the second drive mechanism 88. The second focussing optical element 89 may, as in the present example embodiment, be disposed between the first lens group 86 and the second lens group 87, and may have a convex lens surface facing the first lens group 86 and a concave lens surface facing the second lens group 87, for example. As will be described in more detail below, for operation in the AS imaging mode, the second drive mechanism 88 is controlled to move the second focussing optical element 89 to a reference position P_{ref}, along the optical path P, whereby the focal length of the objective lens system 80 is set to the second focal length FL₂ for imaging the portion of the anterior segment 22 that is of interest. For operation in the PS imaging mode, the second drive mechanism 88 is controlled to move the second focussing optical element 89 in either direction along the optical path P so as to vary the focal length of the objective lens system 80, until the focal length of the objective lens system 80 is set to the first focal length FL₁ for acquiring an in-focus image of the portion of the retina 24 that is of interest.

The scanning element 90 is arranged to perform a scan of a beam of light L_{f1} across the portion of the eye 20 that is of interest via the objective lens system 80 during acquisition of the OCT image 10, and the optics of the objective lens system 80 may, as in the present example embodiment, be arranged such that the scan has a non-rectangular scan pattern scan geometry, i.e. a scan geometry in which the scanned beam of light does not maintain a single direction of propagation as it is swept across the portion of the eye 20. More particularly, the objective lens system 80 may, as in the present example embodiment, be arranged to provide a convergent scan geometry, in which the optical paths followed by the beam as it is scanned across the anterior segment 22 or other portion of the eye 20 converge towards the eye 20. For example, where the convergent scan geometry is a concentric (or "arc") scan geometry, a largely perpendicular angle of incidence on the cornea is maintained. In other example embodiments, however, the objective lens system 80 may be arranged to provide a divergent scan geometry, such as a "sector" scan geometry, in which the beam of light is swept in a divergent fan. It should be noted that the objective lens system 80 may be configured to provide a convergent or other non-rectangular scan geometry using an arrangement of optical elements different from that described herein with reference to Figures 3A and 3B. In yet other example embodiments, the objective lens system 80 may be arranged to provide a rectangular (or "telecentric") scan geometry, in which the scanned beam of light maintains a single direction of propagation as it is swept across the portion of the eye 20.

For further details of how the objective lens system 80 may be configured for imaging the retina 24 and the anterior segment 22 of the eye 20 by selective insertion of the first focussing optical element 83 into the optical path P within the objective lens system 80, as described above, reference is made to US 2022/0079434 A1, the contents of which are incorporated herein by reference in their entirety.

Along with the adjustment of the focal length of the objective lens system 80 that needs to be made when changing between the PS and AS imaging modes, it is also necessary to appropriately change the optical delay between the two arms of the interferometer 30 so that the OCT imaging window can also be disposed at an appropriate position long the optical axis A of the objective lens system 80 that is suitable for imaging the portion of the anterior segment 22 in the AS imaging mode, and for imaging the portion of the retina 24 in the PS imaging mode.

Depending on its location in the interferometer 30, the adjustable optical delay line 50 may be used to adjust the optical path length of the reference arm 32 or the sample arm 34, and thereby an optical delay between the lights in the two arms of the interferometer 30, and consequently also the axial position (along the z-axis shown in Figures 1 and 2) of the measured axial depth range. As noted above and illustrated in Figures 1 and 2, the adjustable optical delay line 50 is provided in the sample arm 34 of the interferometer 30 in the present example embodiment.

The adjustable optical delay line 50 may be of a free-space form, wherein moveable mirrors 52, which may form part of a retroreflector (e.g. a corner cube reflector), for example, are mounted on a motorised linear translation stage which is moveable in accordance with control signals generated by the controller 70 to vary an optical path length of the (preferably collimated) signal light L_{f1}, L_{f2} propagating through the adjustable optical delay line 50. The motorised translation stage may be driven by any appropriate means, for example a piezo transducer in cases where a relatively small travel range is required, or a motor such as a slip-stick motor, an inchworm motor or a DC motor with an encoder in cases where a larger travel range is required.

Motorised translation stages and other mechanisms that are often employed in adjustable optical delay lines tend to introduce some variability in the optical delay which is achieved when the adjustable optical delay line 50 is repeatedly adjusted back to a target setting. This variability depends on the type of optical delay line used, and may be reduced by equipping the optical delay line with an optical interferometer and a control mechanism which uses the interferometer to accurately monitor movements within the delay line and correct for deviations from target values using a feedback loop. However, such solutions add complexity and additional maintenance requirements, as well as cost, and yet fail to account for unwanted changes to the delay between the arms of the OCT imaging instrument's interferometer that may originate outside the optical delay line.

In view of these problems, the controller 70 is arranged to control the adjustable optical delay line 50 to adjust the axial position of the OCT imaging window on the basis of OCT data acquired as the adjustment is being made, using a surface or another portion of the optical element 40 as a datum to place the OCT imaging window at a known position along the optical axis A of the objective lens system 80, at which the portion of the eye 20 is located during its imaging by the ophthalmic OCT imaging instrument 100'. The operation of the controller 70 is described in more detail below with reference to Figures 5 and 6.

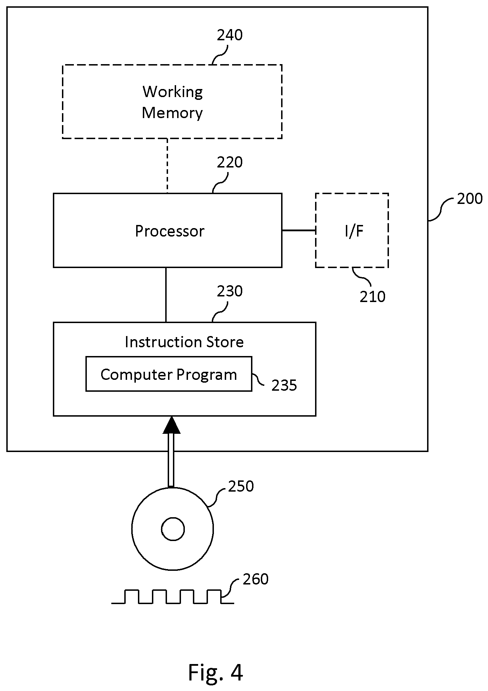

The controller 70 may be provided in any suitable form, for example as a processor 220 of a programmable signal processing hardware 200 of the kind illustrated schematically in Figure 4. The programmable signal processing hardware 200 comprises a communication interface (I/F) 210, for communicating control signals and/or data with the objective lens system 80, the detector 60 and the drive mechanism 85, as described herein. The signal processing hardware 200 further comprises a processor 220 (e.g. a Central Processing Unit, CPU, and/or a Graphics Processing Unit, GPU), and an instruction store 230 storing a computer program 235 comprising the computer-readable instructions which, when executed by the processor 220, cause the processor 220 to perform various functions of the controller 70 described herein. The signal processing hardware 200 may further comprise a working memory 230 (e.g. a random-access memory), which stores information used by the processor 220 during execution of the computer program 235.

The instruction store 230 may comprise a ROM (e.g. in the form of an electrically erasable programmable read-only memory (EEPROM) or flash memory) which is pre-loaded with the computer-readable instructions. Alternatively, the instruction store 230 may comprise a RAM or similar type of memory, and the computer-readable instructions of the computer program 235 can be input thereto from a computer program product, such as a non-transitory, computer-readable storage medium 250 in the form of a CD-ROM, DVDROM, etc. or a computer-readable signal 260 carrying the computer-readable instructions. In any case, the computer program 235, when executed by the processor 220, causes the processor 220 to perform the functions of the controller 70 described herein. More generally, the controller 70 may comprise one or more instances of the computer processor 220 and one or more instances of the memory 230 storing computer-readable instructions which, when executed by the computer processor(s) 220, cause the computer processor(s) 220 to perform some or all of the functions of the controller 70 described herein.

The controller 70 may alternatively be implemented in non-programmable hardware, such as an ASIC, an FPGA or other integrated circuit dedicated to performing the functions of the controller 70 described herein, or a combination of such non-programmable hardware and programmable hardware as described above with reference to Figure 4. The controller 70 may thus be implemented in any computer hardware configured to provide the described functionality.

When the ophthalmic OCT imaging instrument 100' is to be used to image the retina 24 of the eye 20, the controller 70 adjusts the focal length of the objective lens system 80 to the first focal length FL₁, and sets the optical delay between the two arms of the interferometer 30 to a value for imaging the retina 24. The controller 70 may adjust the focal length starting from a nominal (e.g. zero dioptre) focal length, which would be suitable for imaging the fundus of an eye of an average size which is non-myopic, for example. The nominal focal length may alternatively be suitable for imaging the fundus of an eye which has a prescribed level of myopia that is average for a population of interest, for example. The controller 70 may adjust the focal length of the objective lens system 80 from the nominal focal length to the first focal length FL₁ that is suitable for acquiring a focused image of the retina 24 (i.e. an image of the retina 24 having a maximum degree of focus achievable) by controlling the movement of one or more lenses of the objective lens system 80 relative to a remainder of the objective lens system 80, along the optical axis A of the objective lens system 80.

For example, in the implementation of the objective lens system 80 described above with reference to Figures 3A and 3B, the controller 70 controls the first drive mechanism 84 to move the first focussing optical element 83 out of the optical path P through the objective lens system 80, and controls the OCT imaging instrument 100' to acquire images of the retina 24 while controlling the second drive mechanism 88 to move the second focussing optical element 89 along the optical path P, from a starting position corresponding to the nominal focal length mentioned above, thereby adjusting the focal length of the objective lens system 80. The controller 70 may, as in the present example embodiment, analyse acquired OCT data as the adjustment is being made to determine when the focal length of the objective lens system 80 has been adjusted to the first focal length FL₁ for imaging the retina 24. For example, the controller 70 may analyse OCT images (e.g. B-scans) generated by the controller 70 to determine the location, within an image frame of the OCT images, of a predetermined anatomical feature of the retina 24 that is present in the OCT images, such as the surface of the retina or a prescribed retinal layer (e.g. the retinal pigment epithelium, RPE), and can be reliably detected automatically from its characteristic spatial intensity variation, for example. Once the controller 70 determines that the predetermined anatomical feature is at a predetermined location (or with a predetermined range of locations) within the image frame, the controller 70 determines that the focal length of the objective lens system 80 has been adjusted to the first focal length FL₁, and stops adjusting the focal length by movement of the second focussing optical element 89.

The ophthalmic imaging instrument 100 is then ready to image the retina 24. For improved image quality through reduced motion artefacts, gaze fixation is preferably employed to fix the gaze direction of the eye 20 in a gaze direction that is appropriate for the target region of the retina 24 that is to be imaged, e.g. a central gaze direction. To acquire the OCT image 15 of the retina 15, the swept narrowband light source 38' of the OCT imaging instrument 100' illuminates a portion of the retina 24 with the signal light L_{f1} via the objective lens system 80 while the focal length of the objective lens system 80 remains at the first focal length FL₁, and the image-forming device formed by detector 60 and controller 70 images the illuminated portion of the retina 24 as described above. OCT imaging of the retina has then been completed, and the ophthalmic OCT imaging instrument 100' switches from operating in the PS imaging mode to operating in the AS imaging mode.

Before imaging of the anterior segment 22 can begin, the controller 70 is required to adjust the focal length of the objective lens system 80 to the second focal length FL₂, and adjust the optical delay between the two arms of the interferometer 30 to a value suitable for imaging the required portion of the anterior segment 22. The controller 70 therefore controls the objective lens system 80 to sets its focal length to the second focal length FL₂. In the implementation of the objective lens system 80 in Figures 3A and 3B, for example, the controller 70 generates a control signal for controlling the first drive mechanism 84 to move the first focussing optical element 83 into the optical path P passing through the objective lens system 80, and generates a second control signal for controlling the second drive mechanism 88 to move the second focussing optical element 89 to the reference position P_{ref} along the optical path P, such that the focal length of the objective lens system 80 becomes the second focal length FL₂ for imaging the anterior segment 22.

Figure 5 is a flow diagram illustrating a method of generating control signals for controlling the adjustable optical delay line 50 to change the optical delay (preferably from a predetermined value) to a target value for imaging a portion of the eye 20 that is of interest. In the present example embodiment, this method is used to control the adjustable optical delay line 50 to set the optical delay to a target value for imaging the anterior segment 22.

In process S10 of Figure 5, the controller 70 generates a first control signal, S_{D1}, for controlling the adjustable optical delay line 50 to start varying the optical delay, (preferably from the predetermined value). The predetermined value may, as in the present example embodiment, be a value of the optical delay for which the OCT imaging window is within the objective lens system 80, for example between the first lens group 86 and the first focussing optical element 83 shown in Figure 3B, and has been changed by the controller 70 from another value previously used for imaging the retina 24 in the PS imaging mode. The adjustable optical delay line 50 may vary the optical delay in any suitable way, at a constant rate or otherwise, whether continuously or in discrete steps, such that the OCT imaging window moves along the optical axis A of the objective lens system 80, towards the eye 20.

After the adjustable optical delay line 50 has started to vary the optical delay, in process S20 of Figure 5, the controller 70 generates A-scans by processing the electrical signal S generated by the detector 60 using well-known OCT data processing techniques, such that each of the A-scans corresponds to a different value of the optical delay. A sequence of A-scans is thus generated as the adjustable optical delay line 50 is being adjusted, where the A-scans in the sequence image respective portions of the optical path P, and each A-scan added to the sequence images a portion of the optical path P which is closer to the eye 20 than the portions of the path P imaged in the previously acquired A-scans. Each A-scan may form part of a respective B-scan or C-scan, and it will be understood that the following description, which refers to the processing of A-scans, also applies to the processing of B-scans and C-scans acquired as the optical delay is being varied.

In process S30 of Figure 5, the processor 70 processes each A-scan to determine whether A-scan data in the A-scan, or A-scan data that is confined to a predetermined portion (e.g. a central portion) of the A-scan, is indicative of a portion of the optical element 40 having been imaged in the A-scan or the predetermined portion (as the case may be), until a first A-scan comprising A-scan data that is indicative of the portion of the optical element 40 having been imaged in the A-scan or the predetermined portion (as the case may be) has been processed. The controller 7 may make the determination in process S30 of Figure 5 by determining whether the magnitude of any data element or the magnitudes of a prescribed number of sequential data elements in the A-scan (or the predetermined portion thereof, as the case may be) exceeds a predetermined threshold. However, the controller 7 may make the determination in process S30 of Figure 5 in other ways, for example by determining whether the variation of the magnitudes of data elements in the A-scan (or the predetermined portion thereof, as the case may be) fits a prescribed pattern with a goodness-of-fit (as evaluated using any suitable metric, such as the Kolmogorov-Smirnov test or the Chi-squared test, for example) which is above a threshold value. The processing of the first A-scan corresponds to a detection of the portion of the optical element 40 by the controller 70.

The portion of the optical element 40 may be a surface of the optical element 40, which may provide a relatively strong reflection of the signal light L_{f1} and therefore be easier to detect. For example, in the present example embodiment, optical element 40 is a lens forming part of the first lens group 86 and, more specifically, the outermost lens of the lenses in the first lens group 86, which lens is closest to the eye 20, and the portion of the optical element 40 is a surface of the outermost lens of the objective lens system 80, which surface faces the eye 20 during acquisition of the OCT image 10.

In response to the processing of the first A-scan (i.e. the detection of the surface or other portion of the optical element 40), the controller 70 generates, in process S40 of Figure 5, a second control signal, S_{D2}, for controlling the adjustable optical delay line 50 to change the optical delay by a predetermined amount to the target value for imaging the portion of the eye 20, which is the anterior segment 22 in the present example embodiment. The predetermined amount, by which the delay is adjusted, is dependent on an optical path length of the sample light L_{f1} between the anterior segment 22 and the optical element 40 when the eye 20 is disposed at a predetermined position relative to the OCT imaging instrument 100' for acquiring the OCT image 10, and causes the OCT imaging window to move from a position along the optical axis A at which it images the surface of the outermost lens of the objective lens system 80 facing the eye 20, to a position along the optical axis A at which it images the anterior segment 22. When the subject engages with a patient interface of the ophthalmic OCT imaging instrument 100', such a chin rest, forehead rest or the like, and the objective lens system 80 is aligned with the pupil of the eye 20 longitudinally along the optical axis A and transversely (i.e. in directions in the x-y plane perpendicular to the optical axis A) using a pupil alignment module (PAM) employing stereo cameras for range-finding and transverse alignment, for example, the anterior segment 22 will be at a known working distance from the closest surface of the objective lens system 80. This known working distance can be used to determine the predetermined amount by which the optical delay needs to be adjusted to bring it to the target value for imaging the anterior segment 22 of the eye 20. As the optical delay is thus adjusted to the target value on the basis of a datum, which has been found by processing OCT data acquired as the adjustment is being made, and which has a known spatial relationship to the position at which the anterior segment 22 is known to be located during OCT imaging, the optical delay can be accurately set for imaging the anterior segment each time the ophthalmic OCT imaging instrument switches to operating in the AS imaging mode.

It should be noted that the method described above with reference to Figure 5 is applicable not only to the generation of control signals for controlling the adjustable optical delay line 50 to set the optical delay for imaging the anterior segment 22 of the eye 20 when the ophthalmic OCT imaging instrument 100' switches to operating in the AS imaging mode but also to the generation of control signals for controlling the adjustable optical delay line 50 to set the optical delay for imaging the retina 24 when the ophthalmic OCT imaging instrument 100' switches to operating in the PS imaging mode. Furthermore, these techniques are more widely applicable to the control of the optical delay line in the interferometer of any ophthalmic OCT imaging instrument in which the delay provided by the optical delay line is changed to allow the eye to be imaged at different ranges of depth that are not limited to the anterior and posterior segments of the eye, and may, for example, together cover a greater part of the anterior segment than could achieved using a single value for the optical delay.

Further still, the techniques described herein are applicable to the control of the optical delay line in the interferometer of an ophthalmic OCT imaging instrument in which the delay provided by the optical delay line needs to be set for imaging the eye over a single range of depths, which may, for example, cover only the retina or only the anterior segment. The optical delay line in conventional ophthalmic OCT imaging instruments of this kind would not normally require adjustment after the instrument has been commissioned. However, adjusting the optical delay in such ophthalmic OCT imaging instruments prior to imaging, using the techniques described herein, may advantageously allow unwanted changes in the location of the OCT imaging window relative to the ophthalmic OCT imaging instrument, which may be caused by replacement of interferometer components or components becoming misalignment during servicing or repair, for example, to be avoided.

When the controller 70 generates a B-scan or a C-scan of the anterior segment 20, it is beneficial to process the acquired A-scan data using a so-called "de-warping" algorithm, in order to compensate for the refractive index transition at the air/tear interface and the different refractive indices of the various tissues in the anterior segment, and thus map A-scan data values that are indicative of tissue reflectance, to points in space where the originating tissue elements are predicted to have been located (the A-scans themselves only providing information on the reflectance variation along the beam path used to acquire the A-scan). The de-warping algorithm required to generate a geometrically accurate rendering of the anterior segment 22, in which the sizes of anatomical features and angles therebetween can be measured accurately, will depend on the scan geometry of the ophthalmic OCT imaging instrument 100, 100'. The converging scan geometry, which is employed in the present example embodiment, tends to require a de-warping of the OCT image data whose effectiveness is sensitive to the placement of the anterior segment 22 within the OCT imaging window, in other words, the correct positioning of the focal plane of the objective lens system 80 (which has a fixed positional relationship to the converging beam paths) relative to the anterior segment 22 of the eye 20. The same can be said for the diverging (e.g. sector) scan geometry. A placement of the anterior segment 22 away from its optimal position within the OCT imaging window (in the axial (z-axis) direction or along the optical axis A) tends to result in the rendered B-scan or C-scan being distorted, from which reliable distance and angle measurements may be difficult or impossible.

It is therefore preferable, especially where the ophthalmic OCT imaging instrument 100, 100' employs a non-rectangular scan geometry and, in particular, a converging scan geometry, that the OCT imaging window is brought into close alignment (in the z-axis direction) with the anterior segment 22 after the optical delay line 50 has been set for imaging the anterior segment 22, as described above.

The ophthalmic OCT imaging instrument 100' may therefore further comprise a drive mechanism 85, as illustrated in Figure 2, which is operable to move the objective lens system 80 along the optical axis A thereof, towards and away from the eye 20. Furthermore, the controller 70 may, as in the present example embodiment, be further arranged to control the drive mechanism 85, in an optional process S50 in Figure 5, to adjust the position of the objective lens system 80 relative to the eye 20. Process S50 of Figure 5 will now be described in detail with reference to Figure 6.

In process S52 of Figure 6, the controller 70 generates a drive control signal, S_{M}, for controlling the drive mechanism 85 to start moving the objective lens system 80 along the optical axis A. The drive mechanism 85 may move the objective lens system 80 in any suitable way, at a constant rate or otherwise, whether continuously or in discrete steps, such that the objective lens system 80 moves along its optical axis A towards or away from the eye 20. The movement need not be unidirectional, in the sense that the direction of travel of the objective lens system 80 may reverse in case the scleral spur, iris or other predetermined anatomical feature of the anterior segment 22 has not been imaged in process S56 described below before the objective lens system 80 has travelled a predetermined distance in the initial direction of travel. As an alternative to such movement direction reversal, the objective lens system 80 may be backed off after it has been aligned with the pupil by the PAM or the like, to ensure that a movement (in process S52 of Figure 6) in the opposite direction is required to eventually image the predetermined anatomical feature in process S56 of Figure 6.

After the drive mechanism 85 has started to move the objective lens system 80, in process S54 of Figure 6, the controller 70 generates A-scans by processing the electrical signal S generated by the detector 60 such that each of the A-scans corresponds a different distance of the objective lens system 80 from the eye 20. A sequence of A-scans is thus generated as the objective lens system 80 is being moved, where the A-scans in the sequence image respective portions of the optical path P, and each A-scan added to the sequence images a portion of the optical path P which is closer to the eye 20 (or further away from the eye 20, as the case may be) than the portions of the path P imaged in the previously acquired A-scans. Each A-scan may form part of a respective B-scan or C-scan.

In process S56 of Figure 6, the controller processes each A-scan to determine whether A-scan data in the A-scan, or A-scan data that is confined to a predetermined portion (e.g. an end portion) of the A-scan, is indicative of a predetermined anatomical feature in the anterior segment 22 having been imaged in the A-scan or the predetermined portion (as the case may be), until a first A-scan comprising A-scan data that is indicative of the predetermined anatomical feature having been imaged in the A-scan or the predetermined portion (as the case may be) has been processed. The predetermined anatomical feature may, for example, be the apex or other predetermined portion of the cornea of the eye 20, for example.

Where the controller 70 generates B-scans by processing the electrical signal S generated by the detector 60 such that each of the B-scans corresponds a different distance of the objective lens system 80 from the eye 20, in process S56 of Figure 6, the controller 70 may process each B-scan to determine whether B-scan data that is confined to a predetermined range of positions along an axial direction in the B-scan is indicative of a predetermined anatomical feature in the anterior segment 22 having been imaged in the predetermined range, until a first B-scan comprising B-scan data that is indicative of the predetermined anatomical feature having been imaged in the predetermined range of positions along an axial direction in the B-scan has been processed. In this case, the predetermined anatomical feature may, for example, be the scleral spur of the eye 20 or the iris of the eye 20.

In case individual A-scans that do not form part of B-scans are being processed, the controller 70 may make the determination in process S56 of Figure 6 by determining whether the magnitude of any data element or the magnitudes of a prescribed number of sequential data elements in the A-scan (or the predetermined portion thereof, as the case may be) exceeds a predetermined threshold. However, the controller 70 may make the determination in process S56 of Figure 6 in other ways, for example by determining whether the variation of the magnitudes of data elements in the A-scan (or the predetermined portion thereof, as the case may be) fits a prescribed pattern with a goodness-of-fit (as evaluated using any suitable metric, such as the Kolmogorov-Smirnov test or the Chi-squared test, for example) which is above a threshold value.

In case B-scans (or C-scans) are being processed, the controller 70 may make the determination in process S56 of Figure 6 by using a trained machine learning algorithm to locate the predetermined anatomical feature within the B-scan (or C-scan), and determine whether the predetermined anatomical feature is within the predetermined portion of the B-scan (or C-scan). For example, the controller 70 may determine whether the scleral spurs in the B-scan are located within a band extending across a B-scan, in a direction normal to the axial direction in the B-scan, where the width and location of the predetermined band within the B-scan are set to ensure that the anterior segment 22 is appropriately positioned within the frame of the B-scan for the OCT data to be de-warped correctly to produce a de-warped B-scan when the scleral spurs have been moved to within the predetermined band by the movement of the objective lens system 80. The predetermined band may, for example, extend across the middle of the B-scan, and the scleral spur of the eye 20 may be located in the focal plane of the objective lens system 80 when they are imaged within the predetermined band of the B-scan.

The processing of the first A-scan (or the first B-scan, as the case may be) corresponds to a detection of the predetermined anatomical feature by the controller 70. Where individual A-scans (rather than B-scans or C-scans) are being processed, the processing of the first A-scan may correspond to the presence of the apex of the cornea in a predetermined section of the A-scan, whose size and location within the A-scan are set to ensure that the eye 20 is appropriately positioned relative to the objective lens system 80 for the OCT data to be de-warped correctly to produce a de-warped B-scan or C-scan when the apex of the cornea has been brought within the predetermined section by the movement of the objective lens system 80.

In process S58 of Figure 6, in response to the processing of the first A-scan (or the first B-scan or the first C-scan, as the case may be), the processor 70 causes the drive mechanism 85 to stop moving the objective lens system 80, as the eye 20 is then appropriately positioned in relation to the objective lens system 80 for the de-warping algorithm to produce effectively de-warped B-scans or C-scans of the anterior segment 22. The ophthalmic OCT imaging instrument 100' is then ready to image the anterior segment 22. The controller 70 may case the drive mechanism 85 to stop moving the objective lens system 80 in any suitable way, for example by ceasing to generate the drive control signal S_{M} or by providing the drive mechanism 85 with another control signal which instructs the drive mechanism 85 to stop moving the objective lens system 80.

### [Modifications and Variations]

Although the example embodiments described above employ free-space optics, those arrangements are not limiting. In other example embodiments herein, optical fibre-based configurations can be employed, in which optical fibres are used in place of free space to propagate beams of light, and a fibre coupler unit (FCU) may be used in place of the beam splitter described above.

Furthermore, although the example embodiment of Figure 2 is implemented using so-called unbalanced detection, in which the interfering frequency components are directly detected from an optical signal by the photodetector 62, a balanced detection arrangement may be employed in other example embodiments, in which the return lights from the sample arm 34 and the reference arm 32 are combined at a (e.g. 50:50) coupler and the outputs of the coupler are coupled to different optical inputs of a balanced detector.

Further still, although an example of the ophthalmic OCT imaging instrument 100 in the form of an SS-OCT imaging instrument 100' has been described above with reference to Figure 2, this is only an example, and the ophthalmic OCT imaging instrument 100 may take many other forms whose implementations are well-known. The ophthalmic OCT imaging instrument 100 may, for example, be another kind of Fourier-domain OCT (FD-OCT) imaging instrument, such as a spectral-domain OCT (SD-OCT) imaging instrument, or may alternatively be a time-domain OCT (TD-OCT) imaging instrument.

In some example embodiments, the ophthalmic OCT imaging instrument 100, 100' may be operable to acquire retinal images of a different modality than the images of the anterior segment 22, as well as images of the retina 24 and the anterior segment 22 that are of the same modality. For example, the ophthalmic OCT imaging instrument 100, 100' may be further arranged to generate an OCT angiography (OCTA) image of the illuminated portion of the retina 24 by repeatedly detecting an interference signal resulting from an interference between the reference light Lᵣ₂ and the reflected signal L_{f2} in the PS imaging mode, for example by acquiring repeat B-scans of a targeted region of the retina 24. The processor 70 may generate OCTA data representable as the OCTA image by processing the detected interference signals using well-known OCTA data processing techniques, a review of which may be found in the article "Optical Coherence Tomography Angiography" by S. S. Gao et al., Investigative Ophthalmology & Visual Science, Special Issue, Vol. 57, No. 9, OCT27-OCT36, for example.

Moreover, although the OCT imaging instruments of the example embodiments described above are ophthalmic OCT imaging instruments for imaging the eye of a subject in vivo, the techniques described herein are more generally applicable to OCT imaging instruments for imaging other imaging targets/objects, such as the skin of the subject or a sample of any kind, whether biological or non-biological.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the functions of the controller 70, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. An optical coherence tomography, OCT, imaging instrument (100) arranged to acquire an OCT image (10) of an imaging target, the OCT imaging instrument (100) comprising:
an interferometer (30) having a reference arm (32) and a sample arm (34) comprising an optical element (40), the interferometer (30) being arranged to generate an interference light (L_{I}) during use of the OCT imaging instrument (100) by combining a sample light (L_{f2}) that has been scattered by the imaging target and propagates via the optical element (40) during acquisition of the OCT image (10), with a reference light (Lᵣ₂) propagating along the reference arm (32) during acquisition of the OCT image (10);
an adjustable optical delay line (50) controllable to adjust an optical delay between the sample light (L_{f2}) and the reference light (Lᵣ₂);
a detector (60) arranged to detect the interference light (L_{I}) and generate an electrical signal (S) indicative of the detected interference light (L_{I}); and
a controller (70) arranged to control the adjustable optical delay line (50), using the electrical signal (S), to change the optical delay to a target value for imaging the imaging target, by:
controlling the adjustable optical delay line (50) to start varying the optical delay;
after the adjustable optical delay line (50) has started to vary the optical delay, generating A-scans by processing the electrical signal (S) being generated by the detector (60), such that each of the A-scans corresponds to a different value of the optical delay;
processing each A-scan to determine whether A-scan data in a portion of the A-scan is indicative of a portion of the optical element (40) having been imaged in the portion of the A-scan, until a first A-scan comprising A-scan data that is indicative of the portion of the optical element (40) having been imaged in the portion of the A-scan has been processed; and
in response to the processing of the first A-scan, controlling the adjustable optical delay line (40) to change the optical delay by a predetermined amount to the target value for imaging the imaging target, the predetermined amount being dependent on an optical path length of the sample light between the imaging target and the optical element (40) when the imaging target is disposed at a predetermined position relative to the OCT imaging instrument (100) for the OCT image (10) to be acquired.

2. The OCT imaging instrument (100) according to Claim 1, further comprising:
an objective lens system (80) provided in the sample arm (34) of the interferometer (30); and
at least one scanning element (90) arranged to perform a scan of a beam of light (L_{f1}) across the imaging target via the objective lens system (80) during acquisition of the OCT image (10), the objective lens system (80) being arranged such that the scan has a non-rectangular scan geometry.

3. The OCT imaging instrument (100) according to Claim 2, wherein the objective lens system (80) is arranged such that the scan has a converging scan geometry.

4. The ophthalmic OCT imaging instrument (100) according to Claim 2 or Claim 3, wherein the optical element (40) comprises a lens, and the objective lens system (80) comprises a plurality of lenses that includes the lens, the lens being an outermost lens of the plurality of lenses which is closest to the imaging target during acquisition of the OCT image (10).

5. The ophthalmic OCT imaging instrument (100) according to Claim 4, wherein the portion of the optical element (40) is a surface of the outermost lens, which surface faces the imaging target during acquisition of the OCT image (10).

6. The ophthalmic OCT imaging instrument (100) according to any preceding claim, wherein the imaging target comprises an anterior segment (22) of an eye (20), and the OCT imaging instrument (100) is further arranged to acquire an OCT image (15) of a retina (24) of the eye (20), the controller (70) being arranged to control the adjustable optical delay line (50), using the electrical signal (S), to change the optical delay from a predetermined value, which is for imaging the retina (24) of the eye (20), to the target value for imaging the anterior segment (22).

7. The ophthalmic OCT imaging instrument (100) according to Claim 2 or Claim 3, wherein
the imaging target comprises an anterior segment (22) of an eye (20), and the OCT imaging instrument (100) is further arranged to acquire an OCT image (15) of a retina (24) of the eye (20), the controller (70) being arranged to control the adjustable optical delay line (50), using the electrical signal (S), to change the optical delay from a predetermined value, which is for imaging the retina (24) of the eye (20), to the target value for imaging the anterior segment (22), and
the ophthalmic OCT imaging instrument (100) further comprises a drive mechanism (85) operable to move the objective lens system (80) along an axis, towards and away from the eye (20), wherein the controller (70) is further arranged to control the drive mechanism (85), after controlling the adjustable optical delay line (50) to change the optical delay to the target value for imaging the anterior segment (22) of the eye (20), to adjust a position of the objective lens system (80) relative to the eye (20), by:
controlling the drive mechanism (85) to start moving the objective lens system (80) along the axis;
after the drive mechanism (85) has started to move the objective lens system (80), generating A-scans by processing the electrical signal (S) being generated by the detector (60), such that each of the A-scans corresponds a different distance of the objective lens system (80) from the eye (20);
processing each A-scan to determine whether A-scan data in a portion of the A-scan is indicative of a predetermined anatomical feature in the anterior segment (22) having been imaged in the portion of the A-scan, until a first A-scan comprising A-scan data that is indicative of the predetermined anatomical feature having been imaged in the portion of the A-scan has been processed; and
in response to the processing of the first A-scan, controlling the drive mechanism (85) to stop moving the objective lens system (80).

8. The ophthalmic OCT imaging instrument according to Claim 7, wherein
after the drive mechanism (85) has started to move the objective lens system (80), the controller (70) is arranged to generate B-scans of the anterior segment (22) of the eye (20) by processing the electrical signal (S) being generated by the detector (60), such that each of the B-scans corresponds a different distance of the objective lens system (80) from the eye (20),
the predetermined anatomical feature comprises one of a scleral spur of the eye (20), an iris of the eye (20), and a predetermined portion of a cornea of the eye (20),
the controller (70) is arranged to process each B-scan to determine whether B-scan data in the B-scan is indicative of the predetermined anatomical feature having been imaged within a predetermined range of positions along an axial direction in the B-scan, until a first B-scan comprising B-scan data that is indicative of the predetermined anatomical feature having been imaged within the predetermined range of positions has been processed, and
in response to the processing of the first B-scan, the controller (70) is arranged to control the drive mechanism (85) to stop moving the objective lens system (80).

9. A computer-implemented method of generating control signals for controlling an optical coherence tomography, OCT, imaging instrument (100) which is arranged to acquire an OCT image (10) of an imaging target, the OCT imaging instrument (100) comprising:
an interferometer (30) having a reference arm (32) and a sample arm (34) comprising an optical element (40), the interferometer (30) being arranged to generate an interference light (L_{I}) during use of the OCT imaging instrument (100) by combining a sample light (L_{f2}) that has been scattered by the imaging target and propagates via the optical element (40) during acquisition of the OCT image (10), with a reference light (Lᵣ₂) propagating along the reference arm (34) during acquisition of the OCT image (10);
an adjustable optical delay line (50) controllable to adjust an optical delay between the sample light (L_{f2}) and the reference light (Lᵣ₂); and
a detector (60) arranged to detect the interference light (L_{I}) and generate an electrical signal (S) indicative of the detected interference light (L_{I}),
the method comprising generating control signals (S_{D1}, S_{D2}) for controlling the adjustable optical delay line (50) to change the optical delay to a target value for imaging the imaging target, by:
generating (S10) a first control signal (S_{D1}) for controlling the adjustable optical delay line (50) to start varying the optical delay;
after the adjustable optical delay line (50) has started to vary the optical delay, generating (S20) A-scans by processing the electrical signal (S) generated by the detector (60), such that each of the A-scans corresponds to a different value of the optical delay;
processing (S30) each A-scan to determine whether A-scan data in a portion of the A-scan is indicative of a portion of the optical element (40) having been imaged in the portion of the A-scan, until a first A-scan comprising A-scan data that is indicative of the portion of the optical element (40) having been imaged in the portion of the A-scan has been processed; and
in response to the processing of the first A-scan, generating (S40) a second control signal (S_{D2}) for controlling the adjustable optical delay line (50) to change the optical delay by a predetermined amount to the target value for imaging the imaging target, the predetermined amount being dependent on an optical path length of the sample light (L_{f2}) between the imaging target and the optical element (40) when the imaging target is disposed at a predetermined position relative to the OCT imaging instrument (100) for acquiring the OCT image (10).

10. The computer-implemented method according to Claim 9, wherein the OCT imaging instrument (100) further comprises:
an objective lens system (80) provided in the sample arm (34) of the interferometer (30); and
at least one scanning element (90) arranged to perform a scan of a beam of light (L_{f1}) across the imaging target via the objective lens system (80) during acquisition of the OCT image (10), the objective lens system (80) being arranged such that the scan has a non-rectangular scan geometry.

11. The computer-implemented method according to Claim 9 or Claim 10, wherein the imaging target comprises an anterior segment (22) of an eye (20), the OCT imaging instrument (100) is further arranged to acquire an OCT image (15) of a retina (24) of the eye (20), and the generated control signals (S_{D1}, S_{D2}) are for controlling the adjustable optical delay line (50) to change the optical delay from a predetermined value, which is for imaging the retina (24) of the eye (20), to the target value for imaging the anterior segment (22) of the eye (20).

12. The computer-implemented method according to Claim 10, wherein
the imaging target comprises an anterior segment (22) of an eye (20), the OCT imaging instrument (100) is further arranged to acquire an OCT image (15) of a retina (24) of the eye (20), and the generated control signals are for controlling the adjustable optical delay line (50) to change the optical delay from a predetermined value, which is for imaging the retina (24) of the eye (20), to the target value for imaging the anterior segment (22) of the eye (20),
the ophthalmic OCT imaging instrument (100) further comprises a drive mechanism (85) controllable to move the objective lens system (80) along an axis, towards and away from the eye (20), and
the computer-implemented method further comprises, after generating (S40) the second control signal (S_{D2}), generating (S50) at least one drive control signal (S_{M}) for controlling the drive mechanism (85) to adjust a position of the objective lens system (80) relative to the eye (20), by:
generating (S52) a drive control signal (S_{M}) for controlling the drive mechanism (85) to start moving the objective lens system (80) along the axis;
after the drive mechanism (85) has started to move the objective lens system (80), generating (S54) A-scans by processing the electrical signal (S) generated by the detector (60), such that each of the A-scans corresponds a different distance of the objective lens system (80) from the eye (20);
processing (S56) each A-scan to determine whether A-scan data in a portion of the A-scan is indicative of a predetermined anatomical feature in the anterior segment (22) having been imaged in the portion of the A-scan, until a first A-scan comprising A-scan data that is indicative of the predetermined anatomical feature having been imaged in the portion of the A-scan has been processed; and
in response to the processing of the first A-scan, causing (S58) the drive mechanism (85) to stop moving the objective lens system (80).

13. The computer-implemented method according to Claim 12, wherein
after the drive mechanism (85) has started to move the objective lens system (80), B-scans of the anterior segment (22) of the eye (20) are generated by processing the electrical signal (S) generated by the detector (60), such that each of the B-scans corresponds a different position of the objective lens system (80) relative to the eye (20),
the predetermined anatomical feature comprises one of a scleral spur of the eye (20), an iris of the eye (20), and a predetermined portion of a cornea of the eye (20),
each B-scan is processed to determine whether B-scan data in the B-scan is indicative of the predetermined anatomical feature having been imaged within a predetermined range of positions along an axial direction in the B-scan, until a first B-scan comprising B-scan data that is indicative of the predetermined anatomical feature having been imaged within the predetermined range of positions has been processed, and
the drive mechanism (85) is caused to stop moving the objective lens system (80) in response to the processing of the first B-scan.

14. A computer program (235) comprising computer-readable instructions that, when executed by a processor (220), cause the processor (220) to perform a method according to at least one of claims 9 to 13.

15. A computer-readable storage medium (250) storing the computer program (235) according to claim 14.
